# EUROPEAN PATENT APPLICATION

(11) **EP 4 834 343 A1**
(43) Date of publication of application: **04.11.2026**
(21) Application number: 24912986.7
(22) Date of filing: 26.12.2024
(51) Int. Cl.: A61K 31/4745, A61P 25/16, A61P 25/28

(54) **THERAPEUTIC AGENT FOR NEURODEGENERATIVE DISORDER**

(30) Priority: 28.12.2023 JP 2023223448
(71) Applicant: Kissei Pharmaceutical Co., Ltd., Matsumoto-Shi, Nagano 399-8710 (JP)
(72) Inventor: FURIHATA, Takao, Tokyo 112-0002 (JP); SAKUWA, Yoshihiro, Tokyo 112-0002 (JP); OIKAWA, Junichi, Tokyo 112-0002 (JP); UMEZAWA, Yuko, Tokyo 112-0002 (JP)
(74) Representative: Boult Wade Tennant LLP
(86) International application number: PCT/JP2024/046107
(87) International publication number: WO 2025/143100

(57) **Abstract**

An object of the present invention is to provide a medicine that is a non-ergot DA, reduces a risk of somnolence as a side effect, and exhibits an excellent therapeutic effect against neurodegenerative diseases such as PD.

The present invention relates to a pharmaceutical composition for treating a neurodegenerative disease such as Parkinson's disease including: 1-{[(4aR,6R,8aR)-2-amino-3-cyano-8-methyl-4,4a,5,6,7,8,8a,9-octahydrothieno[3,2-g]quinoline-6-yl]carbonyl}-3-[2-(dimethylamino)ethyl]-1-propylurea or a pharmacologically acceptable salt thereof, wherein the compound is orally administered in an amount of 0.25 mg to 2 mg in terms of a free form equivalent value as a daily dose.

## Description

### [Technical Field]

The present invention relates to a medicine that reduces a risk of somnolence as a side effect caused by a non-ergot dopamine agonist and exhibits an excellent therapeutic effect against neurodegenerative diseases such as Parkinson's disease.

More specifically, the present invention relates to a pharmaceutical composition for treating a neurodegenerative disease including: 1-{[(4aR,6R,8aR)-2-amino-3-cyano-8-methyl-4,4a,5,6,7,8,8a,9-octahydrothieno[3,2-g]quinoline-6-yl]carbonyl}-3-[2-(dimethylamino)ethyl]-1-propylurea or a pharmacologically acceptable salt thereof, the compound being orally administered in an amount of 0.25 mg to 2 mg in terms of a free form equivalent value as the amount administered once a day.

### [Background Art]

Parkinson's disease (PD) is a neurodegenerative disease in which the midbrain dopaminergic neurons degenerate and are lost, leading to motor disorders due to a decrease in dopamine, such as resting tremors, rigidity, akinesia, and postural reflex impairments, which progress gradually. Also, non-motor symptoms such as autonomic nervous symptoms and depression preceding the motor disorders, sleep disorders, and dementia concurrently occur.

There has been no fundamental treatment for PD, and symptomatic treatment using drugs for the purpose of alleviating symptoms has mainly been conducted. There has been a report that dopamine agonists (DAs) have a longer half-life in blood as compared with levodopa preparations and develop less wearing-off phenomena and motor complications such as dyskinesia observed in the case of levodopa preparations. The DAs are categorized into an ergot type and a non-ergot type depending on differences in chemical structures, and non-ergot DAs (such as pramipexole and ropinirole) have widely been used in clinical practice because there have been reports that ergot DAs (such as cabergoline and pergolide) develop a valvular heart disease as a serious side effect.

However, concerns have been raised regarding clinical applications of non-ergot DAs as well, and "somnolence" has been listed as a well-known adverse event of the non-ergot DAs (NPL 1). There has been a report that non-ergot DAs have higher risks of somnolence as a side effect as compared with ergot DAs (NPL 2) and existing non-ergot DAs have higher risks of somnolence as a side effect as compared with placebo (NPL 3 and 4) from meta-analysis of clinical tests.

Although a decrease in amount, discontinuation, and the like of the DAs has also been considered for somnolence, there is insufficient evidence regarding this. A risk of deterioration of motor symptoms accompanying the decrease in amount has also been pointed out (NPL 1). It has been difficult to achieve both maintenance of a sufficient therapeutic effect and reduction of the risk of side effects by the non-ergot DAs.

As described above, development of a novel DA that is a non-ergot DA, reduces a risk of somnolence as a side effect, and exhibits effectiveness that is equivalent to or higher than that of existing DAs is desired.

PTL 1, 2, and 3 describe 1-{[(4aR,6R,8aR)-2-amino-3-cyano-8-methyl-4,4a,5,6,7,8,8a, 9-octahydrothieno[3,2-g]quinoline-6-yl]carbonyl}-3-[2-(dimethylamino)ethyl]-1-propylurea represented by Formula (I) below (hereinafter, referred to as Compound 1). Also, a succinate of Compound 1 is mentioned in PTL 3. PTL 1, 2, and 3 describe that an octahydrothienoquinoline derivative containing Compound 1 or a pharmacologically acceptable salt thereof has a dopamine D₂ receptor agonistic effect and can be used as a preventive or therapeutic agent for PD, a restless legs syndrome, or hyperprolactinemia. PTL 1, 2, and 3 describe that it is possible to manufacture an agent for oral administration such that the octahydrothienoquinoline derivative or the pharmacologically acceptable salt thereof is administered within a range of about 0.1 mg to about 300 mg. However, none of the literatures describes that Compound 1 or the pharmacologically acceptable salt thereof reduces the risk of somnolence and exhibits an excellent effect of ameliorating neurodegenerative diseases such as PD.

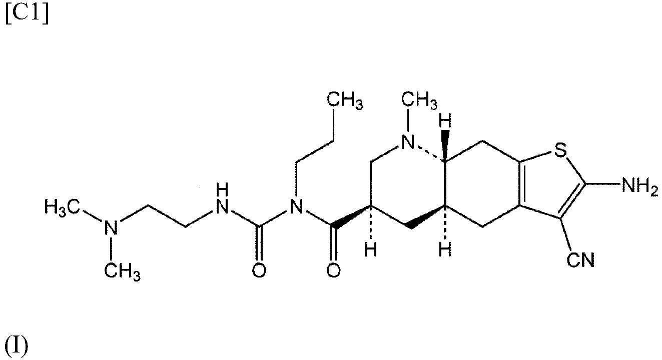

### [Citation List]

### [Patent Literature]

[PTL 1] WO 2012/124649
[PTL 2] Japanese Patent No. 6177061
[PTL 3] WO 2022/009815

### [Non Patent Literature]

[NPL 1] Parkinson's Disease Clinical Practice Guidelines 2018, p. 34 to 57, 217 to 219
[NPL 2] Stowe R, "The Cochrane database of systematic reviews", 2008, Vol. 2, CD006564
[NPL 3] Wu, Chunxiao, "Frontiers in Aging Neuroscience", 2022, Vol. 14, 831884
[NPL 4] Chen, Xiang-Ting, "Frontiers in neurology", 2023, Vol. 14, 1183823

### [Summary of Invention]

### [Technical Problem]

An object of the present invention is to provide a medicine that is a non-ergot DA, reduces a risk of somnolence as a side effect, and exhibits an excellent therapeutic effect against neurodegenerative diseases such as PD.

### [Solution to Problem]

The present inventors have discovered as a result of intensive studies to solve the above problem, that surprisingly, using Compound 1 or the pharmacologically acceptable salt thereof not only has an obvious effect of ameliorating neurodegenerative diseases such as PD but also can reduce a risk of somnolence as a side effect.

In other words, the present invention relates to [1] to [5] below and the like.
[1] A pharmaceutical composition for treating a neurodegenerative disease comprising 1-{[(4aR,6R,8aR)-2-amino-3-cyano-8-methyl-4,4a,5,6,7,8,8a,9-octahydrothieno[3,2-g]quinoline-6-yl]carbonyl}-3-[2-(dimethylamino)ethyl]-1-propylurea or a pharmacologically acceptable salt thereof, wherein the compound is orally administered in an amount of 0.25 mg to 2 mg in terms of a free form equivalent value as a daily dose.
[2] The pharmaceutical composition according to the above [1], wherein the pharmacologically acceptable salt is a succinate of 1-{[(4aR,6R,8aR)-2-amino-3-cyano-8-methyl-4,4a,5,6,7,8,8a,9-octahydrothieno[3,2-g]quinoline-6-yl]carbonyl}-3-[2-(dimethylamino)ethyl]-1-propylurea.
[3] The pharmaceutical composition according to the above [1] or [2], wherein the daily dose is 0.25 mg to 1.5 mg in terms of a free form equivalent value, and the pharmaceutical composition is administered once a day.
[4] The pharmaceutical composition according to any one of the above [1] to [3], wherein the daily dose is 0.25 mg for initial administration and is 0.5 mg to 2 mg for subsequent administration in terms of a free form equivalent value, and the pharmaceutical composition is administered once a day.
[5] The pharmaceutical composition according to any one of the above [1] to [4], wherein the neurodegenerative disease is Parkinson's disease.

Also, as one embodiment, the present invention relates to a method for treating a neurodegenerative disease including: administering, to a patient, a necessary amount of pharmaceutical composition comprising 1-{[(4aR,6R,8aR)-2-amino-3-cyano-8-methyl-4,4a,5,6,7,8,8a,9-octahydrothieno[3,2-g]quinoline-6-yl]carbonyl}-3-[2-(dimethylamino)ethyl]-1-propylurea or a pharmacologically acceptable salt thereof, wherein the compound is orally administered in an amount of 0.25 mg to 2 mg in terms of a free form equivalent value as a daily dose.

Moreover, as one embodiment, the present invention relates to use of 1-{[(4aR,6R,8aR)-2-amino-3-cyano-8-methyl-4,4a,5,6,7,8,8a,9-octahydrothieno[3,2-g]quinoline-6-yl]carbonyl}-3-[2-(dimethylamino)ethyl]-1-propylurea or a pharmacologically acceptable salt thereof for manufacturing a pharmaceutical composition for treating a neurodegenerative disease, wherein the compound is orally administered in an amount of 0.25 mg to 2 mg in terms of a free form equivalent value as a daily dose.

### [Advantageous Effects of Invention]

The pharmaceutical composition of the present invention exhibits an excellent therapeutic effect against neurodegenerative diseases such as PD and reduces a risk of somnolence as a side effect.

### [Brief Description of Drawings]

[Fig. 1]

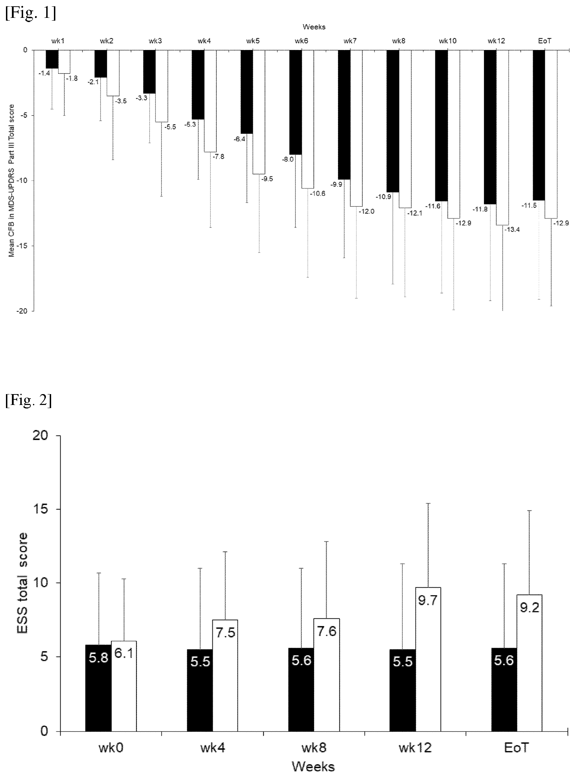

   Fig. 1 shows an effect of ameliorating motor symptoms of PD patients. The vertical axis represents the amount of change from a baseline (CFB) of the Movement Disorder Society-Unified Parkinson's Disease Rating Scale (MDS-UPDRS) Part III total score. The horizontal axis represents an evaluation timing. "wk 1, 2, 3, 4, 5, 6, 7, 8, 10, 12" means 1 week, 2 weeks, 3 weeks, 4 weeks, 5 weeks, 6 weeks, 7 weeks, 8 weeks, 10 weeks, and 12 weeks from the start of administration, respectively. "EoT (End of Treatment)" means the timing of treatment phase final evaluation. In the drawing, the black bar graph indicates values of Compound 2 administration group, and the white bar graph indicates values of pramipexole hydrochloride hydrate extended-release tablet (PPX-LA) administration group (an average value ± a standard deviation).
[Fig. 2]
   Fig. 2 shows somnolence evaluation for the PD patients. The vertical axis represents Epworth Sleepiness Scale (ESS) total scores. The horizontal axis represents evaluation timings. "wk 0" means the timing of the start of administration, and "wk 4, 8, 12" means 4 weeks, 8 weeks, and 12 weeks from the start of administration, respectively. "EoT" means the timing of treatment phase final evaluation. In the drawing, the black bar graph indicates values of Compound 2 administration group, and the white bar graph indicates values of the PPX-LA administration group (an average value ± a standard deviation).
[Fig. 3]

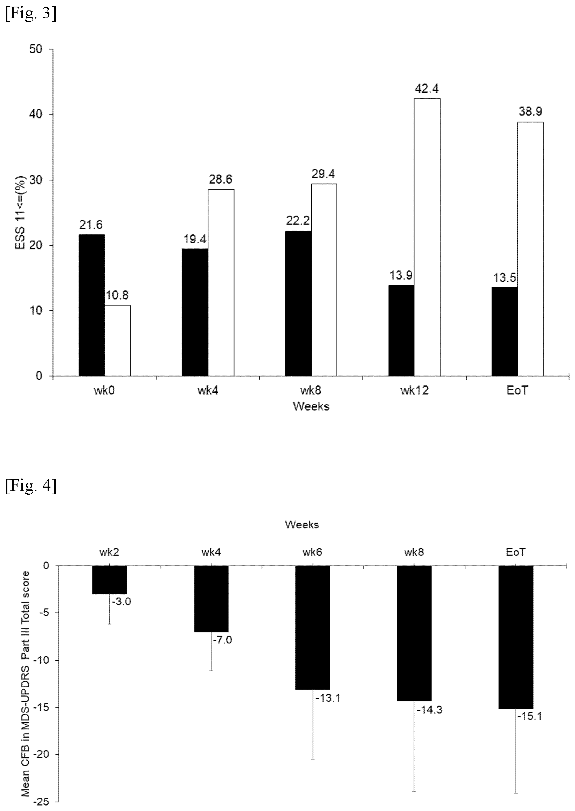

   Fig. 3 shows somnolence evaluation for PD patients. The vertical axis represents proportions of subjects with ESS total scores of equal to or greater than 11 points. The horizontal axis represents evaluation timings. "wk 0" means the timing of the start of administration, and "wk 4, 8, 12" means 4 weeks, 8 weeks, and 12 weeks from the start of administration, respectively. "EoT" means the timing of treatment phase final evaluation. In the drawing, the black bar graph indicates values of Compound 2 administration group, and the white bar graph indicates values of the PPX-LA administration group.
[Fig. 4]
   Fig. 4 shows effects of ameliorating motor symptoms of PD patients. The vertical axis represents amounts of change from the baseline of the MDS-UPDRS Part III total score. The horizontal axis represents evaluation timings. "wk 2, 4, 6, 8" means 2 weeks, 4 weeks, 6 weeks, and 8 weeks from the start of administration, respectively. "EoT" means the timing of treatment phase final evaluation. In the drawing, the black bar graph indicates values of Compound 2 administration group (an average value ± a standard deviation).
[Fig. 5]

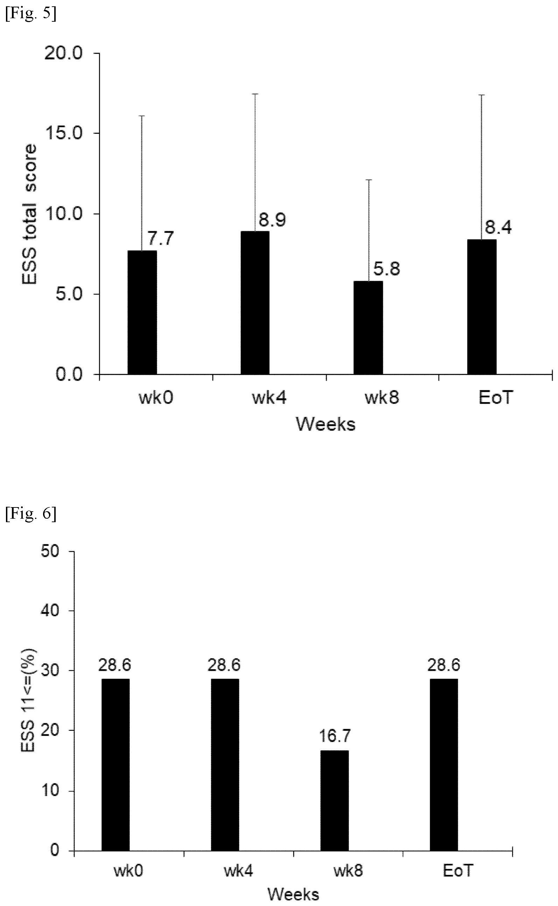

   Fig. 5 shows somnolence evaluation of PD patients. The vertical axis represents ESS total scores. The horizontal axis represents evaluation timings. "wk 0" means the timing of the start of administration, and "wk 4, 8" means 4 weeks and 8 weeks from the start of administration, respectively. "EoT" means the timing of treatment phase final evaluation. In the drawing, the black bar graph indicates values of Compound 2 administration group (an average value ± a standard deviation).
[Fig. 6]
   Fig. 6 shows somnolence evaluation for PD patients. The vertical axis represents proportions of subjects with ESS total scores of equal to or greater than 11 points. The horizontal axis represents evaluation timings. "wk 0" means the timing of the start of administration, and "wk 4, 8" means 4 weeks and 8 weeks from the start of administration, respectively. "EoT" means the timing of treatment phase final evaluation. In the drawing, the black bar graph indicates values of Compound 2 administration group.

### [Description of Embodiments]

Hereinafter, embodiments of the present invention will be described in more detail.

In the present invention, unless otherwise specified, respective terms have the following meanings.

In the present invention, Compound 1 means "1-{[(4aR,6R,8aR)-2-amino-3-cyano-8-methyl-4,4a,5,6,7,8,8a,9-octahydrothieno[3,2-g]quinoline-6-yl]carbonyl}-3-[2-(dimethylamino)ethyl]-1-propylurea". Note that Compound 1 and "1-{[(4aR,6R,8aR)-2-amino-3-cyano-8-methyl-4H,4aH,5H,6H,7H,8H,8aH,9H-thieno[3,2-g]quinoline-6-yl]carbonyl}-3-[2-(dimethylamino)ethyl]-1-propylurea" described in PTL 1 are the same compound.

In the present invention, Compound 1 can also be a pharmacologically acceptable salt thereof in accordance with the conventional method, as needed. Examples of such a salt include acid addition salts with mineral acids such as hydrochloric acid, hydrobromic acid, hydroiodic acid, sulfuric acid, nitric acid, phosphoric acid, acid addition salts with organic acids such as formic acid, acetic acid, trifluoroacetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid, and the like, salts with inorganic bases such as lithium salts, sodium salts, potassium salts, calcium salts, magnesium salts, and the like, salts with organic bases such as triethylamine, piperidine, morpholine, lysine and the like. Preferable examples include succinate. In the present invention, examples of succinate of Compound 1 also include a salt of Compound 1 described in PTL 3 and succinic acid at a ratio of 1:1 and a salt of Compound 1 described in PTL 3 and succinic acid at a ratio of 2:3 (hereinafter, this may be referred to as "2/3 succinate of Compound 1"), both of which are included.

In the present invention, "Compound 1 or the pharmacologically acceptable salt thereof" also includes a solvate with a solvent that is pharmacologically acceptable as a medicine, such as water, ethanol and the like.

The compound 1 of the present invention and the pharmacologically acceptable salt thereof can be manufactured by known methods. For example, Compound 1 of the present invention can also be manufactured in accordance with WO 2012/124649 (PTL 1), and succinate of Compound 1 can be manufactured by the method described in WO 2022/009815 (PTL 3) or a method similar thereto, respectively.

The pharmaceutical composition of the present invention may be used in various dosage forms depending on usage. Examples of such dosage forms include agents for oral administration such as powders, granules, fine granules, dry syrups, tablets, and capsules.

The pharmaceutical composition of the present invention is prepared using Compound 1 or the pharmacologically acceptable salt thereof as an active ingredient and at least one pharmaceutical additive. These pharmaceutical compositions can also be prepared by being appropriately mixed, diluted or dissolved with a pharmaceutical additive by a pharmaceutically known method depending on a dosage form. Examples of such a pharmaceutical additive include excipients such as lactose, lubricants such as magnesium stearate, disintegrants such as carboxymethyl cellulose, binders such as hydroxypropyl methylcellulose, surfactants such as macrogol, foaming agents such as sodium bicarbonate, dissolution aids such as cyclodextrin, sour agents such as citric acid, stabilizing agents such as sodium edetate, pH modifiers such as phosphates, and the like.

As another aspect, the pharmaceutical composition of the invention is useful for treatment of neurodegenerative diseases. Although the neurodegenerative diseases are not limited as long as they are characterized by the insidious onset, progression of cell death, loss of functions, examples thereof include multiple system atrophy, progressive supranuclear palsy, corticobasal degeneration, familial dementia, Alzheimer's disease, PD, Huntington's disease, multiple sclerosis, Lewy body dementia, mild cognitive impairment, retinal neurodegeneration, amyotrophic lateral sclerosis, frontotemporal dementia, and the like in the present invention.

As another embodiment, the pharmaceutical composition of the invention is useful as a therapeutic agent for PD and has an effect of ameliorating PD-associated motor disorders (such as tremor, rigidity, bradykinesia, and axial symptoms) and/or reducing a risk of incidence of non-motor symptoms (such as somnolence, gastrointestinal symptoms, and psychiatric symptoms).

Also, as one aspect, the pharmaceutical composition of the present invention can reduce a risk of somnolence as a side effect in a treatment phase of PD in accordance with the aspect, and can thus be used for PD patients, from which sufficient effects cannot be obtained because it is not possible to increase the amounts of existing non-ergot DAs due to somnolence as their side effect.

In the present invention, the effect of ameliorating PD-associated motor disorders can be evaluated using scores known to those skilled in the art as an evaluation measure for the PD motor symptoms. Examples thereof include the MDS-UPDRS scores, the postural instability gait disorder (PIGD) scores, the Schwab & England ADL scales, and the like.

The MDS-UPDRS scores, which are one of the most representative indicators, consist of four parts (that is, Part I to Part IV), and cognitive function and mood disorders, daily life behavior, motor symptoms, motor complications are evaluated in Part I, Part II, Part III, and Part IV, respectively. In the evaluation of amelioration of motor symptoms, scores in Part III are particularly used, and lower scores from the baseline indicate higher amelioration effects.

In the present invention, a daily dose of active ingredient (Compound 1 or the pharmacologically acceptable salt thereof) for adults can be determined within a range of 0.25 mg to 2 mg in terms of a free form equivalent value for oral administration. Also, it is also possible to use the active ingredient while appropriately increasing or decreasing the amount thereof within the range of 0.25 mg to 2 mg in terms of a free form equivalent value in accordance with doctor's judgment based on ages, body weights, degrees of disease, developed states of side effects, and the like of patients, and the daily dose can also be administered once or several times as needed.

As the usage and dose of the pharmaceutical composition of the present invention, 0.25 mg of Compound 1 or the pharmacologically acceptable salt thereof in terms of a free form equivalent value may be orally administered once a day as an initial dose. The present pharmaceutical composition can suitably be incrementally increased in the range of 0.25 mg to 2 mg after the initial administration to define the maintenance amount.

As an aspect, Compound 1 or the pharmacologically acceptable salt thereof (for example, Compound 2, which will be described later) is used in the escalating dosage in which Compound 1 or the pharmacologically acceptable salt thereof is administered at a dose that does not produce clinically meaningful efficacy and side effects (0.25 mg or less in terms of a free form equivalent value as a daily dose of oral administration, for example) as an initial dose, the dose is increased by 0.25 mg or 0.5 mg once or twice or more for each specific follow-up period (one week, two weeks, or a period of combining them, for example), and a dose (0.5 mg to 2 mg, 0.5 mg to 1.5 mg, 0.5 mg to 1 mg, 0.75 mg to 2 mg, 0.75 mg to 1.5 mg, 0.75 mg to 1 mg, 1 mg to 2 mg, 1 mg to 1.5 mg, 1.5 mg to 2 mg, 0.5 mg, 0.75 mg, 1.0 mg, 1.5 mg, or 2.0 mg, for example, in terms of a free form equivalent value as a daily dose) exhibiting effectiveness (the effect of ameliorating motor disorders accompanying neurodegenerative diseases, for example) without developing side effects (such as somnolence, digestive symptoms, and mental symptoms) is defined as a maintenance dose.

As an aspect, the amount administered once a day may be started from 0.25 mg of Compound 1 or the pharmacologically acceptable salt thereof (for example, Compound 2, which will be described later) in terms of a free form equivalent value in oral administration as an initial dose (at 0 weeks), the amount administered once a day may be set to 0.5 mg at a first week, the amount administered once a day may be increased by 0.5 mg every week thereafter, and 0.5 mg to 2 mg thereof may be administered as a maintenance dose. Note that the gradual increase phase may be increased or decreased depending on doctor's judgment and administration may be made by increasing or decreasing the amount administered once a day within the range of 0.25 mg to 2 mg.

As an aspect, the amount administered once a day may be started from 0.25 mg of compound 1 or the pharmacologically acceptable salt thereof (for example, Compound 2, which will be described later) in terms of a free form equivalent value in oral administration as an initial dose (at 0 weeks), the amount administered once a day may be increased by 0.25 mg every two weeks thereafter, and 0.5 mg to 2 mg thereof may be administered as a maintenance dose. Note that the gradual increase phase may be increased or decreased depending on doctor's judgment and administration may be made by increasing or decreasing the amount administered once a day within the range of 0.25 mg to 2 mg.

As one aspect, the amount administered once a day is started from 0.25 mg of compound 1 or the pharmacologically acceptable salt thereof (for example, Compound 2, which will be described later) in terms of a free form equivalent value in oral administration as an initial dose (at 0 weeks), the amount administered once a day is set to 0.5 mg at the first week, and the amount administered once a day is set to 1 mg at the second week. Thereafter, the amount administered once a day may be increased by 0.5 mg every two weeks, and 0.5 mg to 2 mg thereof may be administered as a maintenance dose. Note that the gradual increase phase may be increased or decreased depending on doctor's judgment and administration may be made by increasing or decreasing the amount administered once a day within the range of 0.25 mg to 2 mg.

As an aspect, the pharmaceutical composition of the present invention can exhibit an excellent therapeutic effect for PD and can reduce the risk of somnolence as a side effect, which is one of the side effects of non-ergot DAs. In the present invention, the reduction of the risk of somnolence as a side effect also includes maintaining the risk of somnolence as a side effect, not worsening the risk of somnolence as a side effect, and the like.

In the present invention, "the risk of somnolence as a side effect" of the non-ergot DAs means a risk of developing somnolence in the daytime, which is a safety issue of the non-ergot DAs. The degree of "the reduction of the risk of somnolence as a side effect" achieved by the pharmaceutical composition of the present invention can also be evaluated, for example, by ESS total scores. ESS is a self-managed questionnaire, and is a measure for subjects to evaluate their daytime sleepiness and doze propensity by answering eight questions. The ESS scores (sums of scores for eight items; 0 points to 3 points for each item) range from 0 to 24, and a normal range is 10 points or less. The higher the ESS scores, the higher the average sleep trend or daytime sleepiness in daily lives of the subjects (epworthsleepinessscale.com), and scores that are equal to or higher than 11 points are evaluated as pathological hypersomnia (diagnosis of narcolepsy, treatment guideline item). The effect of "reducing the risk of somnolence as a side effect" achieved by the pharmaceutical composition of the present invention can also be evaluated by, for example, a decrease in incidence rate compared to the developed states of adverse events and side effects by other non-ergot DAs, or a decrease in incidence rate and a decrease in degree of somnolence compared to the incidence frequency of somnolence during the daytime before administration of the pharmaceutical composition of the invention.

### [Examples]

Although the present invention will be described in more detail below on the basis of examples, the present invention is not limited to the content. In the examples, 2/3 succinate of Compound 1 (which will be referred to as Compound 2) was used as an active ingredient of test drugs. The amounts administered (doses) mean doses in free forms (in terms of free form equivalent values) unless otherwise specified.

### Example 1

### Clinical Test 1 Targeted on PD Patients

### 1. Test Method

Effectiveness, safety, and pharmacokinetics when Compound 2 was administered to 75 PD patients with no combined use of levodopa in the amount administered once a day gradually increased in a range of 0.25 mg to 1 mg for 12 weeks were examined by the randomized open-label test method using pramipexole as a comparative drug. Doses and administration methods are shown in Table 1.

Oral administration of Compound 2 in a daily dose of 0.25 mg once a day was started, and the oral administration was conducted once a day while the amount was increased by one stage (0.25 mg as a daily dose) in the order of 0.5 mg, 0.75 mg, and 1 mg as a daily dose unless criteria for not increasing the amount (such as a case where an adverse event with a causal relationship with the investigational drug was recognized and a case where a principal investigator or a sub-investigator determined that there was some safety problem) were met at the timing of 2 weeks, 4 weeks, and 6 weeks of administering the investigational drug. In a case where the criteria for not increasing the amount were met and it was determined to be possible to continue the investigation, the same dose was continuously administered. Note that even in the case where the same dose was continuously administered, the amount was increased when the criteria for not increasing the amount were not met at the next point of increasing the amount (at 4 weeks and 6 weeks). Hereinafter, this group will be referred to as Compound 2 administration group.

Oral administration of pramipexole hydrochloride hydrate extended-release tablet (PPX-LA) in a daily dose of 0.375 mg once a day was started, and the oral administration was conducted once a day while the amount was increased by one stage (0.375 mg or 0.75 mg as a daily dose) in the order of 0.75 mg, 1.5 mg, 2.25 mg, 3 mg, 3.75 mg, and 4.5 mg as a daily dose unless criteria for not increasing the amount were met at the timing of 1 week, 2 weeks, 3 weeks, 4 weeks, 5 weeks, and 6 weeks of administering the investigational drug. In a case where the criteria for not increasing the amount were met and it was determined to be possible to continue the investigation, the same dose was continuously administered. Note that even in the case where the same dose was continuously administered, the amount was increased when the criteria for not increasing the amount were not met at the next point of increasing the amount (at 2 weeks, 3 weeks, 4 weeks, 5 weeks, and 6 weeks). Hereinafter, this group will be referred to as the PPX-LA administration group.

**[Table 1]**

| Administration group | Observation phase | | Treatment phase (12 weeks) | | | | | | | | | | | | | | | | | |
|---|---|---|---|---|---|---|---|---|---|---|---|---|---|---|---|---|---|---|---|---|
| | | | Gradual increase phase (8 weeks) | | | | | | | | | | | | | | | Maintenance phase (4 weeks) | | |
| wk -2 | | wk 0 | | wk 1 | | wk 2 | | wk 3 | | wk 4 | | wk 5 | | wk 6 | | wk 7 | wk 8 | | wk 10 | wk 12 |
| Compound 2 | - | | 0.25 mg/day | | | | 0.5 mg/day | | | | 0.75 mg/day | | | | 1 mg/day | | | 1 mg/day | | |
| PPX-LA | - | | 0.375 mg/day | | 0.75 mg/day | | 1.5 mg/day | | 2.25 mg/day | | 3 mg/day | | 3.75 mg/day | | 4.5 mg/day | | | 4.5 mg/day | | |

### 2. Evaluation Items Related to Effectiveness and Safety

As an evaluation item for effectiveness, measurement values, summary statistics of the amounts of change from the baseline (at 0 weeks), and the like were evaluated for the total scores of MDS-UPDRS scores and total scores of each part. As for the amounts of change in MDS-UPDRS Prat III total scores, the larger amount of change (lower scores) indicates further amelioration.

As evaluation items related to safety, ESS total scores, adverse event developed states, side effect developed states, and the like were evaluated.

### 3. Results

The gradual increase states and the maintenance doses of Compound 2 were as shown in Table 2 below.

**[Table 2]**

| Group | Daily dose | Treatment period | | | | | | | | | |
|---|---|---|---|---|---|---|---|---|---|---|---|
| | | Up-titration | | | | | | | | Maintenance | |
| | | wk 1 | wk 2 | wk 3 | wk 4 | wk 5 | wk 6 | wk 7 | wk 8 | wk 10 | wk 12 |
| Compound 2 (37 cases) | Total | 37 | 37 | 37 | 36 | 36 | 36 | 36 | 36 | 36 | 36 |
| | 0.25 mg | 37 | 37 | 2 | 2 | - | - | - | - | - | - |
| | 0.5 mg | | | 35 | 34 | 2 | 2 | - | - | - | - |
| | 0.75 mg | | | | | 34 | 34 | 2 | 3 | 3 | 3 |
| | 1 mg | | | | | | | 34 | 33 | 33 | 33 |
| PPX-LA (37 cases) | Total | 37 | 37 | 37 | 36 | 34 | 34 | 34 | 33 | 33 | 33 |
| | 0.375 mg | 37 | 1 | - | - | - | - | - | - | - | - |
| | 0.75 mg | | 36 | 2 | 2 | 1 | 1 | 1 | 1 | 1 | 1 |
| | 1.125 mg | | | | | | | | | | 1 |
| | 1.5 mg | | | 35 | 4 | 4 | 4 | 4 | 4 | 4 | 4 |
| | 2.25 mg | | | | 30 | 3 | 2 | 4 | 4 | 3 | 3 |
| | 3 mg | | | | | 26 | 4 | 2 | 2 | 2 | 2 |
| | 3.75 mg | | | | | | 23 | 4 | 5 | 7 | 7 |
| | 4.5 mg | | | | | | | 19 | 17 | 16 | 15 |

The MDS-UPDRS Part III total scores decreased over time with the gradual increase in doses (Fig. 1). The amounts of change in MDS-UPDRS Part III total scores at the treatment phase final evaluation from the baseline (average values ± a standard deviation) were -11.5 ± 7.6 for Compound 2 administration group and -12.9 ± 6.7 for the PPX-LA administration group, and both groups exhibited amelioration of conditions.

The ESS total scores (average values ± a standard deviation) were 5.8 ± 4.9 at 0 weeks and 5.6 ± 5.7 at the treatment phase final evaluation for Compound 2 administration group, and no variations were recognized. For the PPX-LA administration group, the ESS total scores were 6.1 ± 4.2 at 0 weeks and 9.2 ± 5.7 at the treatment phase final evaluation, and an obvious increase was recognized (Fig. 2). The proportions of subjects with ESS total scores of 11 points (the cut off reference for pathological hypersomnia) or more were 21.6% (8/37 cases) at 0 weeks and 13.5% (5/37 cases) at the time of the treatment phase final evaluation for Compound 2 administration group, and were 10.8% (4/37 cases) at 0 weeks and 38.9% (14/36 cases) at the treatment phase final evaluation for the PPX-LA administration group (Fig. 3).

The incidence rates of side effects during the investigational drug administration period were 13.5% (5/37 cases) for Compound 2 administration group and 67.6% (25/37 cases) for the PPX-LA administration group. Among them, the number of developed side effects related to somnolence, mental symptoms, and digestive symptoms are shown in Table 3. The values related all these side effects were low for Compound 2 administration group, and particularly, the development rates of "somnolence" were 2.7% (1/37 cases) for Compound 2 administration group and 29.7% (11/37 cases) for the PPX-LA administration group.

**[Table 3]**

| No. of Adverse drug reactions | | Compound 2(n = 37) | | PPX-LA (n = 37) | |
|---|---|---|---|---|---|
| | | n | % | n | % |
| Total | | 5 | 13.5 | 25 | 67.6 |
| | Somnolence | 1 | 2.7 | 11 | 29.7 |
| | Sudden onset of sleep | 0 | 0.0 | 1 | 2.7 |
| | Hallucination, visual | 0 | 0.0 | 3 | 8.1 |
| | Hallucination, auditory | 0 | 0.0 | 2 | 5.4 |
| | Anxiety | 0 | 0.0 | 2 | 5.4 |
| | Inappropriate affect | 0 | 0.0 | 2 | 5.4 |
| | Insomnia | 1 | 2.7 | 0 | 0.0 |
| | Nausea | 0 | 0.0 | 6 | 16.2 |
| | Vomiting | 0 | 0.0 | 1 | 2.7 |
| | Abdominal discomfort | 0 | 0.0 | 2 | 5.4 |
| | Constipation | 3 | 8.1 | 1 | 2.7 |

As a result of Example 1, Compound 2 administration group had lower incidence rates of adverse events and side effects compared to the PPX-LA administration group, and also had a lower risk of developing side effects, such as daytime somnolence, which was a safety problem of the existing non-ergot DAs. This showed that Compound 2 exhibited an obvious effect of ameliorating motor symptoms of PD within the dose range from 0.25 mg to 1 mg as a daily dose and was able to reduce the risk of somnolence as a side effect while being a non-ergot DA.

### Example 2

### Clinical Test 2 Targeted on PD Patients

### 1. Test Method

Effectiveness, safety, and pharmacokinetics when Compound 2 was orally administered to 7 PD patients with no combined use of levodopa in a daily dose within a range of 0.25 mg to 2 mg once a day and the doses were gradually increased for 8 weeks were examined by the randomized open-label test method. Doses and administration methods are shown in Table 4.

Oral administration of Compound 2 in a daily dose of 0.25 mg once a day was started, and the oral administration was conducted once a day while the amount was increased by one stage (0.25 mg or 0.5 mg as the amount administered once a day) in the order of 0.5 mg, 1 mg, 1.5 mg, and 2 mg as a daily dose unless criteria for not increasing the amount were met at the timing of 1 week, 2 weeks, 4 weeks, and 6 weeks of administering the investigational drug. In a case where the criteria for not increasing the amount were met and it was determined to be possible to continue the investigation, the same dose was continuously administered. Note that even in the case where the same dose was continuously administered, the amount was increased when the criteria for not increasing the amount were not met at the next point of increasing the amount (at 2 weeks, 4 weeks, and 6 weeks).

**[Table 4]**

| Observation phase | | Treatment phase (8 weeks) | | | | | | | | | |
|---|---|---|---|---|---|---|---|---|---|---|---|
| wk -2 | wk 0 | | wk 1 | | wk 2 | | wk 4 | | wk 6 | | wk 8 |
| - | | 0.25 mg/day | | 0.5 mg/day | | 1 mg/day | | 1.5 mg/day | | 2 mg/day | |

### 2. Evaluation Items Related to Effectiveness and Safety

As evaluation items regarding effectiveness and safety, items similar to those in Example 1 were evaluated.

### 3. Results

The gradual increase states of Compound 2 were as shown in Table 5 below.

**[Table 5]**

| Group | Daily dose | Treatment period | | | | |
|---|---|---|---|---|---|---|
| | | Up-titration | | | | |
| | | wk 1 | wk 2 | wk 4 | wk 6 | wk 8 |
| | Total | 7 | 7 | 7 | 7 | 7 |
| | 0.25 mg | 7 | - | - | - | - |
| Compound 2 (7 cases) | 0.5 mg | | 7 | - | - | - |
| | 1 mg | | | 7 | - | - |
| | 1.5 mg | | | | 7 | 1 |
| | 2 mg | | | | | 6 |

The MDS-UPDRS Part III total scores decreased over time with the gradual increase in doses (Fig. 4). The amounts of change in MDS-UPDRS Part III total scores at the treatment phase final evaluation from the baseline (average values ± a standard deviation) were -15.1 ± 9.0.

Also, the ESS total scores (average values ± a standard deviation) were 7.7 ± 8.4 at 0 weeks and 8.4 ± 9.0 at the time of the treatment phase final evaluation, and no significant variations were recognized (Fig. 5). The proportions of subjects with the ESS total scores of 11 points or more were 2 cases out of 7 cases (28.6%) at 0 weeks and 2 cases out of 7 cases (28.6%) at the time of the treatment phase final evaluation (Fig. 6).

Although "hallucination" and "nausea" were observed in 1 case out of 7 cases as side effects during the investigational drug administration period, the degrees of all side effects were mild, and "somnolence" was not observed.

According to the results of Example 2, Compound 2 exhibited an obvious effect of ameliorating motor symptoms of PD even within the daily dose range of 0.25 mg to 2 mg, and no significant concern regarding safety due to the increase in daily dose up to 2 mg was recognized.

### Example 3

### Clinical Test 3 Targeted on PD Patients

### 1. Test Method

Effectiveness, safety, and pharmacokinetics when Compound 2 was administered to early-stage PD patients with no combined use of levodopa in a daily dose gradually increased within a range of 0.25 mg to 2 mg for 17 weeks were examined by the randomized double-blind test method.

Oral administration of Compound 2 was started from a daily dose of 0.25 mg, the daily dose was appropriately gradually increased within a range of 0.25 mg to 2 mg in accordance with doctor's judgment unless the criteria for not increasing the amount were not met at the point of increasing the amount, and the maintenance amount was determined. In a case where the criteria for not increasing the amount were met and it was determined to be possible to continue the investigation, the same dose was continuously administered. Note that even in the case where the same dose was continuously administered, the amount was increased when the criteria for not increasing the amount were not met at the next point of increasing the amount.

### 2. Evaluation Items Related to Effectiveness and Safety

As an evaluation item related to effectiveness, changes of the MDS-UPDRS Part II + III total score from the baseline (first day) to the 17 week were evaluated.

As evaluation items related to safety, ESS total scores, adverse event developed states, side effect developed states, and the like were evaluated.

The purpose of Example 3 was to examine effectiveness and safety in each maintenance amount.

### Example 4

### Clinical Test 4 Targeted on PD Patients

### 1. Test Method

Effectiveness and safety when Compound 2 was administered to PD patients with combined use of levodopa in a daily dose gradually increased within a range of 0.25 mg to 2 mg for 17 weeks were examined by the randomized double-blind test method.

It was confirmed that the target patients satisfied the following selection criteria.

Patients diagnosed with Parkinson's disease on the basis of Parkinson's disease society brain bank clinical diagnostic criteria of Parkinson's Disease Society of the United Kingdom

Patients using levodopa or levodopa combinations and with motor complications such as wearing-off phenomena or dyskinesia or with no sufficient effects of levodopa recognized therefrom

Patients with revised Hoehn & Yahr severity classification (ON time) of 2 to 4 levels

Patients with MDS-UPDRS Part II (ON time) total scores of 2 points or more and with MDS-UPDRS Part III (ON time) total scores of 12 points or more

Patients using levodopa or levodopa combinations and with constant dosages/doses for 4 weeks prior to the start of the screening phase

Oral administration of Compound 2 was started from a daily dose of 0.25 mg, the amount was by one stage (0.25 mg/day or 0.5 mg/day) in the order of 0.5 mg/day, 1 mg/day, 1.5 mg/day, and 2 mg/day in accordance with doctor's judgment unless criteria for not increasing the amount were met at 1 week, 2 weeks, 3 weeks, and 4 weeks of the treatment phase, and the maintenance dose was determined. In a case where the criteria for not increasing the amount were met and it was determined to be possible to continue the investigation, the same dose was continuously administered. Note that even in the case where the same dose was continuously administered, the amount was increased when the criteria for not increasing the amount were not met at the next point of increasing the amount.

**[Table 6]**

| Administration group | Observation phase | | Treatment phase (17 weeks) | | | | | | | | | | | | | |
|---|---|---|---|---|---|---|---|---|---|---|---|---|---|---|---|---|
| | | | Gradual increase phase (5 weeks) | | | | | | | | | | Maintenance phase (12 weeks) | | | |
| wk -2 | | Day 1 | | wk 1 | | wk 2 | | wk 3 | | wk 4 | | wk 5 | | wk 9 | wk 13 | wk 17 |
| Compound 2 | - | | 0.25 mg/day | | 0.5 mg/day | | 1 mg/day | | 1.5 mg/day | | 2 mg/day | | | | | |
| Placebo group | - | | Placebo | | | | | | | | | | | | | |

### 2. Evaluation Items Related to Effectiveness and Safety

### Effectiveness Evaluation Item

MDS-UPDRS Part II + III (ON time) total score
Total scores of each MDS-UPDRS Part I, II, III, and IV (ON time)
OFF time (a time in which Parkinson's symptoms were relatively mild (patients were able to freely move, patients were able to relatively easily move on their own) since levodopa that was being taken worked)
Following ON time (a time in which Parkinson's symptoms were severe (patients were not able to move or were not able to easily move) since levodopa that was being taken did not work):
   ON time with no dyskinesia
   ON time with dyskinesia that did not cause any problems in daily life
   ON time with no dyskinesia that caused problems in daily life
   ON time with dyskinesia that caused problems in daily life
   Summary index of PDQ-39
   Total score of PDSS-2
   Revised Hoehn & Yahr severity classification and the like

### Safety Evaluation Item

Adverse event and side effect
ESS total score and the like

### [Industrial Applicability]

The pharmaceutical composition of the present invention is very useful as a therapeutic agent for neurodegenerative diseases such as Parkinson's disease.

## Claims

1. A pharmaceutical composition for treating a neurodegenerative disease comprising
1-{[(4aR,6R,8aR)-2-amino-3-cyano-8-methyl-4,4a,5,6,7,8,8a,9-octahydrothieno[3,2-g]quinoline-6-yl]carbonyl}-3-[2-(dimethylamino)ethyl]-1-propylurea or a pharmacologically acceptable salt thereof,
wherein the compound is orally administered in an amount of 0.25 mg to 2 mg in terms of a free form equivalent value as a daily dose.

2. The pharmaceutical composition according to claim 1, wherein the pharmacologically acceptable salt is a succinate of 1-{[(4aR,6R,8aR)-2-amino-3-cyano-8-methyl-4,4a,5,6,7,8,8a,9-octahydrothieno[3,2-g]quinoline-6-yl]carbonyl}-3-[2-(dimethylamino)ethyl]-1-propylurea.

3. The pharmaceutical composition according to claim 1, wherein the daily dose is 0.25 mg to 1.5 mg in terms of a free form equivalent value, and the pharmaceutical composition is administered once a day.

4. The pharmaceutical composition according to claim 1, wherein the daily dose is 0.25 mg for initial administration and is 0.5 mg to 2 mg for subsequent administration in terms of a free form equivalent value, and the pharmaceutical composition is administered once a day.

5. The pharmaceutical composition according to claim 1, wherein the neurodegenerative disease is Parkinson's disease.
